# EUROPEAN PATENT APPLICATION

(11) **EP 0 878 543 A1**
(43) Date of publication of application: **18.11.1998**
(21) Application number: 97909690.6
(22) Date of filing: 30.10.1997
(51) Int. Cl.: C12N 15/12, C12P 21/02, C12Q 1/68, C07K 16/18

(54) **cDNA FRAGMENTS OF GENE CAUSATIVE OF SPINOCEREBELLAR ATAXIA TYPE 2**

(30) Priority: 30.10.1996 JP 304059/96
(71) Applicant: SRL, INC., Tachikawa-shi, Tokyo 190 (JP)
(72) Inventor: TSUJI, Shoji, Niigata 950-21 (JP); SANPEI, Kazuhiro, Niigata-shi Niigata 950 (JP)
(74) Representative: Kiddle, Simon John
(86) International application number: JP9703946
(87) International publication number: WO9818920

(57) **Abstract**

A sequence-determined cDNA fragment of the causative gene of SCA2 is disclosed. The cDNA fragment according to the present invention comprises a nucleic acid region encoding an amino acid sequence shown in SEQ ID NO: 1 (provided that the number of repeat units of Gln from the 166th to 188th amino acid varies between 15 and 100).

## Description

### TECHNICAL FIELD

The present invention relates to cDNA fragments of the causative gene of spinocerebellar ataxia type 2 (hereinafter also referred to as 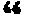 SCA2 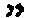 ), proteins encoded thereby, antibodies corresponding to the proteins, and antisense nucleic acids of the above-mentioned cDNA fragments.

### BACKGROUND ART

SCA2 is an autosomal dominant, neurodegenerative disorder that affects the cerebellum and other areas of the central nervous system.

It has recently been discovered that the causative genes of 6 neurodegenerative diseases including dentatorubral-pallidoluysian atrophy (DRPLA) have more CAG repeats than the normal genes. That is, the numbers of CAG repeats in the causative genes of the neurodegenerative diseases are 37 to 100, while those in the normal genes are less than 35.

It has been suggested that the causative gene of SCA2 has an increased number of CAG repeats (Trottier, Y. et al. *Nature*, 378, 403-406 (1995)). However, since the causative gene of SCA2 has not been identified, and since its nucleotide sequence has not been determined, SCA2 cannot be diagnosed by a genetic assay.

### DISCLOSURE OF THE INVENTION

An object of the present invention is to provide a sequence-determined cDNA fragment of the causative gene of SCA2. Another object of the present invention is to provide a protein produced by the causative gene of SCA2. Still another object of the present invention is to provide an antibody specific to the above-mentioned protein, which antibody is useful for therapy and diagnosis of SCA2. Still another object of the present invention is to provide an antisense of the causative gene of SCA2, which is useful for therapy of SCA2.

The present inventors intensively studied to discover a *Tsp* E1 fragment with a size of 2.5 kb in which the number of CAG triplet is increased only in SCA2 patients, and partial sequence thereof was determined. Human cDNA library was screened using as probes the oligonucleotides that respectively hybridize with the regions between which the CAG triplet repeats are interposed, and a cDNA fragment which hybridizes with both of these two probes was cloned. Using this cDNA fragment as a probe, human cDNA library was screened and a plurality of cDNA fragments which hybridize with the probe were cloned. Sequencing the cDNA fragments revealed that these cDNA fragments overlap with each other. To sequence the 5'-end and 3'-end regions, RACE (rapid amplification of cDNA ends) was performed. Further, to sequence the 5'-end region, RT-PCR was performed, thereby succeeding in sequencing the full length of the cDNA of the causative gene of SCA2.

That is, the present invention provides a nucleic acid fragment comprising a nucleic acid region encoding an amino acid sequence shown in SEQ ID NO: 1 (provided that the number of repeat units of Gln from the 166th to 188th amino acid varies between 15 and 100). The present invention also provides a protein having an amino acid sequence encoded by the nucleic acid fragment according to the present invention. The present invention further provides an antibody which undergoes antigen-antibody reaction with the above-mentioned protein. The present invention still further provides an antisense nucleic acid having a size of not less than 15 bp, which hybridizes with a mRNA transcribed from the nucleic acid fragment according to the present invention so as to inhibit translation thereof.

By the present invention, a sequence-determined cDNA fragment of the causative gene of SCA2 was provided. The protein encoded by the nucleic acid fragment according to the present invention may be used for therapy of SCA2 and may be used as an immunogen for preparing an antibody useful for therapy and diagnosis of SCA2. Further, since the nucleotide sequence of the causative gene of SCA2 was determined by the present invention, antisense to this gene may now be designed. Still further, by the present invention, a recombinant vector comprising the nucleic acid fragment according to the present invention which is incorporated in an expression vector that can express a desired gene in human body, which recombinant vector can express the nucleic acid fragment in human body, as well as a method for expressing the nucleic acid fragment according to the present invention comprising introducing the recombinant vector into human body. Thus, the present invention is thought to largely contribute to the therapy and diagnosis of SCA2.

### BRIEF DESCRIPTION OF THE DRAWINGS

Fig. 1 shows the nucleotide sequence of the cDNA fragment according to the present invention together with the amino acid sequence encoded thereby, which nucleotide sequence was determined in the Examples of the present invention.

Fig. 2 shows the continuation of Fig. 1.

Fig. 3 shows the continuation of Fig. 2.

Fig. 4 shows the continuation of Fig. 3.

Fig. 5 is a pedigree chart of the SCA2 patients who donated the genomic DNAs used in the Examples.

Fig. 6 shows the sizes, positions and restriction sites of the genomic DNA fragments Tsp1 and Tsp2, and SCA2 cDNA obtained in the Examples of the present invention, as well as the size and position of each of the obtained cDNA fragment.

Fig. 7 shows the distribution of the numbers of the CAG repeat units in the normal and SCA2 genes, which were measured by using the (CAG)₅₅ probe.

### BEST MODE FOR CARRYING OUT THE INVENTION

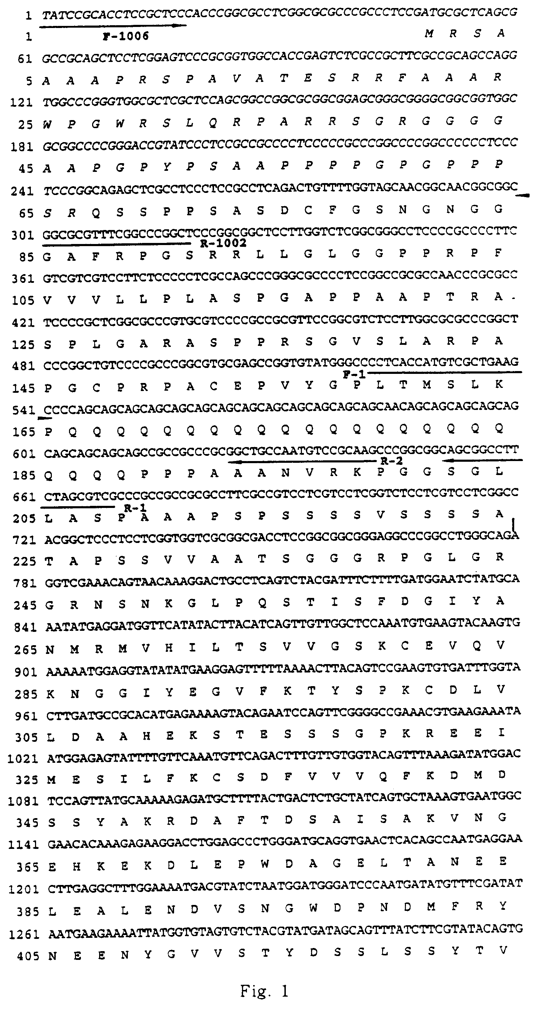

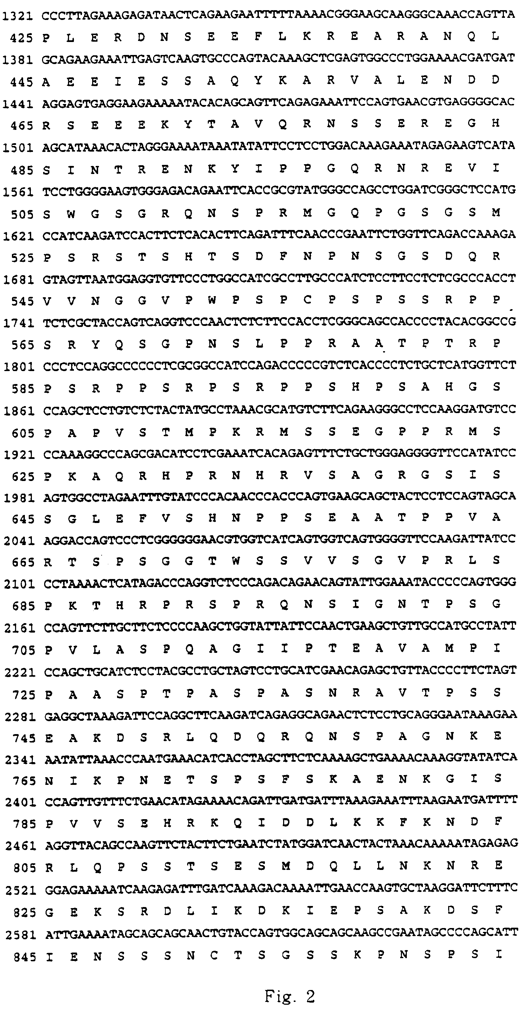

As described above, the nucleic acid fragment according to the present invention comprises the nucleic acid region encoding the amino acid sequence shown in SEQ ID NO: 1 in the SEQUENCE LISTING, provided that the number of repeating units of Gln from the 166th to the 188th amino acid varies between 15 and 100. The number of this repeat units is 15 to 25 in normal individuals and 35 to 100 in SCA2 patients. As is well-known, due to degeneration, there are a plurality of codons encoding one amino acid, and any nucleic acids which encode the amino acid sequence shown in SEQ ID NO: 1 are included within the scope of the present invention. The nucleotide sequence actually determined in the Examples below is shown in SEQ ID NO:1 and in Figs. 1-4. The way how the nucleotide sequence was determined and the fact that the cDNA having this nucleotide sequence is the cDNA of the causative gene of SCA2 are detailed in the Examples below.

The nucleic acid fragment according to the present invention may be cloned by the method detailed in the Examples below. Further, since the nucleotide sequence of the nucleic acid fragment according to the present invention was determined by the present invention, the nucleic acid fragment may be cloned by utilizing amplification by PCR using human cDNA library as a template, or by hybridization using the PCR product as a probe. In cases where it is difficult to amplify the nucleic acid fragment by a single PCR, the nucleic acid fragment may be divided into a plurality of regions and the PCR products may be ligated by a conventional method so as to clone the nucleic acid fragment.

By incorporating the nucleic acid fragment according to the present invention into the multicloning site of a commercially available expression vector, and by transforming host cells with the obtained recombinant vector, the protein encoded by the nucleic acid fragment may be produced. Host-vector systems for expressing an arbitrary gene in a host cell is well-known in the art and a number of host-vector systems are commercially available. Those skilled in the art may easily express the nucleic acid fragment according to the present invention so as to produce the protein using such a commercially available host-vector System. Such a well-known method is described in, for example, D. M. Glover, DNA Cloning Volume III a practical approach, 1987, IRL Press.

For example, according to a conventional method, the nucleic acid fragment according to the present invention may be amplified by the long PCR (LA PCR) (Hiroyuki MUKAI, PROTEIN, NUCLEIC ACID, ENZYME, Vol.41, No.5, 585-594, 1996) using primers in which restriction sites are introduced, and the amplified product is digested with a restriction enzyme. The digested amplified product is then inserted into the multicloning site of a commercially available plasmid vector pGEX (PHARMACIA) and ligated to obtain a recombinant vector, followed by transforming *E. coli* DH5α (GIBCO BRL) by the conventional calcium chloride method. Transformants are then selected based on the drug resistance (ampicillin resistance) and the protein encoded by the causative gene of SCA2 may be recovered from the transformants. In cases where this vector is used, the desired protein is obtained as a fusion protein with GST (Glutathion S-Transferase), so that the protein may easily be detected using a commercially available anti-GST antibody (PHARMACIA).

Since the active protein produced by the gene of normal individuals (the number of CAG repeats is 15 to 25) is thought to have the normal function, SCA2 may be treated or alleviated by administering the normal protein to SCA2 patients. However, since SCA2 is autosomal dominant, it is necessary to simultaneously block the causative gene of SCA2 of the patient with the antisense described below.

By immunizing an animal with the above-described protein or an antigenic fragment thereof, an antibody specific to the protein or the fragment thereof may be recovered from the animal by a conventional method. The antibody may be a polyclonal antibody or a monoclonal antibody, and the monoclonal antibody may be prepared by a well-known method.

It is theoretically difficult to construct an antibody using the polyglutamine chain encoded by the CAG repeat, which antibody has varying reactivities with the polyglutamine chain depending only on the lengths of the polyglutamine chain. However, by using the full length protein which was clarified by the present invention, an antibody which recognizes the stereoscopic difference between the patient's protein and the normal protein may be prepared. Further, by virtue of the present invention, since an antibody may be prepared by using a region of the SCA2 product, which is common to the normal individuals and the patients, a control is available in any assay system using an antibody specific to the protein according to the present invention. Using such an antibody, a simple assay such as plate method may be attained by a conventional method.

An affinity column may be prepared by immobilizing the antibody according to the present invention on agarose gel (e.g., Sephadex (trademark) of PHARMACIA), polystyrene beads or the like and by packing the resultant in a column. By passing a body fluid (blood, serum, spinal fluid or the like) through the affinity column, the protein produced by the causative gene of SCA2 of the patient may be obtained. By eluting the protein and by measuring the molecular weight thereof, diagnosis of SCA2 may be attained. This is because that the protein of a SCA2 patient has a molecular weight larger than that of the protein of a normal individual because the number of CAG repeat units of the SCA2 causative gene in the patient is larger than that of the gene in the normal individual.

Possible gene therapies include the method by which production of the abnormal SCA2 gene product is stopped and the method by which normal SCA2 gene product is introduced. The former method includes blocking of translation of mRNA using an antisense. The latter method includes administration of the SCA2 gene product prepared *in vitro*, and introduction of the nucleic acid fragment into the cells. Since in patients suffering from SCA2 caused by extension of the CAG repeat, the abnormal protein is dominant, it is thought that the desired effect may be obtained by simultaneously performing both of the above-mentioned methods. In this case, the antisense is designed such that it hybridizes with a region not affecting the activity of the SCA2 product, and the region is removed from the normal SCA2 gene to be introduced, thereby assuring that the antisense does not inhibit the production of the normal protein. By designing the antisense and the normal SCA2 gene as mentioned above, the antisense and the SCA2 gene may be incorporated in the same vector and the vector may be introduced into cells.

The antisense nucleic acid according to the present invention hybridizes with the mRNA transcribed from the nucleic acid fragment according to the present invention, and has a size of preferably not less than 15 bp and not more than the full length of the coding region of the nucleic acid fragment, more preferably not less than 50 bp and not more than the full length of the coding region of the nucleic acid fragment. Although the antisense nucleic acid preferably has a nucleotide sequence completely complementary to the entire mRNA or a part thereof, transcribed from the nucleic acid fragment according to the present invention, those having homologies to the degree that they hybridize with the mRNA *in vivo* are within the scope of the present invention. By administering the antisense according to the present invention to an SCA2 patient, the causative gene of SCA2 may be blocked. By administering the above-mentioned protein from a healthy individual to the patient under this condition, SCA2 may be treated or alleviated. The dose of the antisense nucleic acid may be appropriately selected depending on the conditions of the patient, and may usually be 0.001 mmol to 1000 mmol per day per 1 kg of bodyweight.

Known methods for introducing the gene into the body include methods in which Retrovirus or Adenovirus is used as a vector (Yasuhiro SETOGUCHI, Experimental Medicine, Vol.12, No.15 (extra edition) 1994, pp.114-121; Hiromi KANEGAE et al., Experimental Medicine, Vol.12, No.15 (extra edition) 1994, pp.34-40), methods using liposomes, and methods using fusion liposomes (membrane-fused liposome and the like). Among these, since Adenovirus expresses the introduced gene without proliferation of the target cells, the method utilizing Adenovirus is most appropriate as the method for introducing the nucleic acid into nervous system. More particularly, E3 and a part of E1a of the DNA of wild type Adenovirus type 5 are removed and the nucleic acid in double-stranded form is introduced into the virus together with an expression unit such as a promoter. The recombinant virus vector is proliferated in 293 cells expressing E1a and E1b genes, originated from human fetal kidney to prepare a virus liquid, and the virus liquid is inoculated. The virus genome incorporated into the nucleus of the cell exists outside the chromosomes without replication. Since the virus genome is not lost by cell division in nerve cells, expression of normal SCA2 gene may be maintained for 1 to 3 months.

The present invention will now be described more concretely by way of examples thereof. It should be noted that the present invention is not restricted to the following examples.

### Example 1 Preparation of (CAG)₅₅ Probe

A genomic DNA segment of DRPLA gene containing a CAG repeat with 55 repeat units was amplified from the genomic DNA of a patient with DRPLA (Koide, R. et al., Nature Genet., 6, 9-13 (1994)) and was subcloned into a plasmid vector, pT7Blue T (*p-2093*). The *p-2093* plasmid contains the (CAG)₅₅ and the flanking sequences. That is, the plasmid contains the sequence of 5'-CAC CAC CAG CAA CAC CAA (CAG)₅₅ CAT CAC GGA AAC TCT GGG CC-3'. Using a pair of oligonucleotides 5'-CAC CAC CAG CAA CAG CAA CA-3' and 5'-biotin-GGC CCA GAG TTT CCG TGA TG-3', PCR was performed in a total volume of 16 µl containing 10 mM Tris-HCl, pH8.3, 50 mM KCl, 1.5 mM MgCl₂, 2M N,N,N-trimethylglycine, 0.1 mM TTP, 0.1 Mm dCTP, 0.1 mM dGTP, 9.25 MBq of [α-³²P]dATP (222 TBq/mmol), 0.5 µM each of the two primers, 0.3 ng of plasmid DNA (*p-2093*) and 2.0 U of Taq DNA polymerase (Takara Shuzo, Kyoto, Japan). After an initial 2-min. denaturation at 94°C, PCR was performed for 30 cycles consisting of 1-min. denaturation at 94°C, 1-min. annealing at 54°C and 3-min. extension at 72°C, followed by a final extension at 72°C for 10 min.

A single-stranded (CAG)₅₅ probe was isolated using streptavidin-coated magnetic beads (Dynabeads M-280, Streptavidin;Dynal AS, Oslo, Norway) on which 20 µl of streptavidin is coated. That is, after washing of the PCR products immobilized on the magnetic beads with 40 µl of a solution containing 5 mM Tris-HCl (pH 7.5), 0.5 mM EDTA and 1 M NaCl, the non-biotinylated strand containing the radio-label was separated from the biotinylated strand by incubation in 50 µl of 0.1 M NaOH for 10 min. The resultant supernatant was directly added to the hybridization solution described below.

Incidentally, using the single-stranded (CAG)₅₅ probe prepared as described above, Southern blot analysis was carried out on the androgen receptor genes containing 9, 22, 43 and 51 CAG repeat units, respectively. As a result, the (CAG)₅₅ probe strongly hybridized with the genes having 43 and 51 CAG repeats units, respectively, but scarcely hybridized with the gene having 22 CAG repeat units, and did not hybridize at all with the gene having 9 CAG repeat units (K. Sanpei et al., Biochemical and Biophysical Research Communications, Vol.212, No.2, 1995, pp.341-346). Thus, by using this probe, hybridization may be selectively attained only with DNAs containing a number of (e.g., 35 or more) CAG repeat units if the hybridization conditions are appropriately selected.

### (2) Determination of Nucleotide Sequence of SCA2 Gene

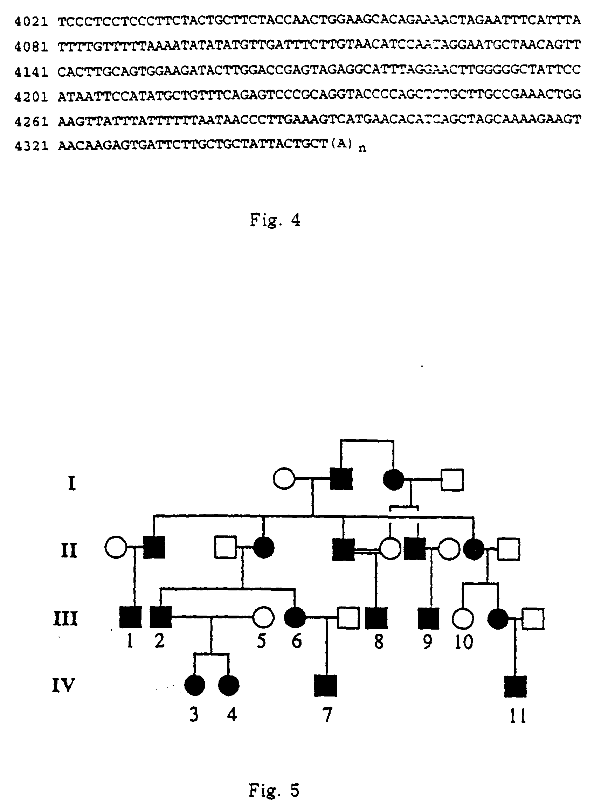

Fig. 5 shows a pedigree chart of SCA2 patients. In this pedigree chart, males are represented by squares and females are represented by circles. SCA2 patients are represented by black squares or circles, and unaffected persons are represented by white squares or circles.

High-molecular-weight genomic DNA (15 µg) was digested with 100 U of *TspE*I (Toyobo, Osaka, Japan), electrophoresed through 0.8% agarose gels and transferred to nitrocellulose membranes. The membranes were hybridized with the (CAG)₅₅ probe described above. Hybridization was performed in a solution containing 2.75 x SSPE (1 x SSPE=150 mM NaCl, 10 mM NaH₂PO₄, 1 mM EDTA), 50% formamide, 5 x Denhardt's solution, 100 ng/ml of sheared salmon sperm DNA and the (CAG)₅₅ probe (6 x 10⁶ cpm/ml) at 62°C for 18 hours. After the hybridization, the membranes were washed with 1 x SSC (150 mM NaCl, 15 mM sodium citrate) containing 0.5% SDS at 65°C for 0.5 hours. The membranes were autoradiographed for 16 hours to Kodak Bio Max MS film at -70°C using an MS intensifying screen.

As a result, 2.5 kbp *TspE*I fragment hybridized with the probe was detected only in all of the SCA2 patients.

Genomic DNA (270 µg) from an SCA2 patient (individual 7 in Fig. 5) was digested by *TspE*I and subjected to agarose gel electrophoresis. Genomic DNA fragments including the 2.5 kb *TspE*I fragment were cloned into an *Eco*RI-cleaved λZAPII vector. The genomic library was screened using the (CAG)₅₅ probe under the hybridization condition described above. A genomic clone, *Tsp*-1, containing an expanded CAG repeat was isolated.

After removal of the probe, the above-described genomic library was screened again using the *Tsp-1* as a probe, which was labeled by the random priming. Hybridization was carried out in a solution containing 5 x SSC, 1 x Denhardt's solution, 10% dextran sulfate, 20 mM sodium phosphate, 400 µg/ml human placental DNA and the *Tsp-1* probe at 42°C for 18 hours. After the hybridization, the membranes were washed finally in 0.1 x SSC - 0.1% SDS at 52°C for 0.5 hours. The membranes were autoradiographed for 24 hours to Kodak Bio Max MS films at -70°C using an MS intensifying screen. As a result, a genomic clone, *Tsp-2*, originated from a normal allele was isolated.

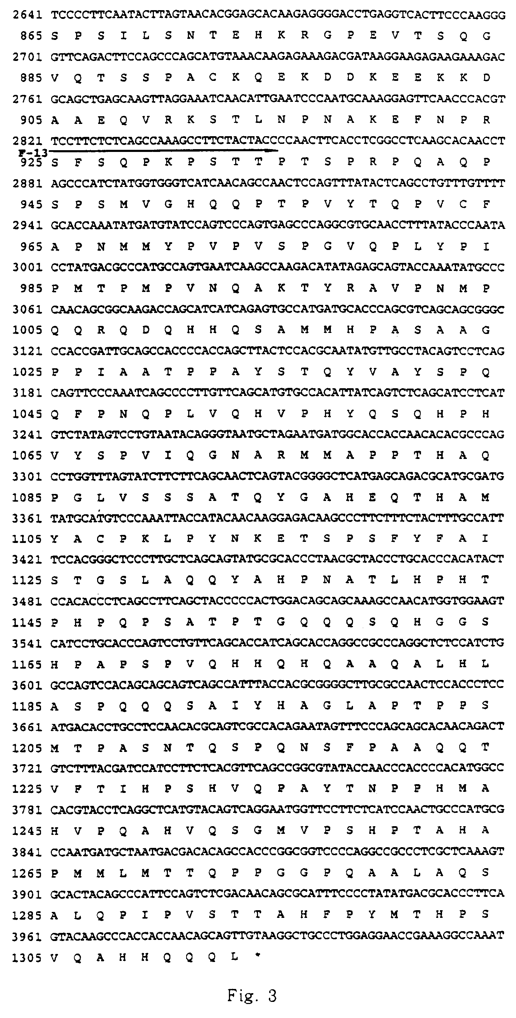

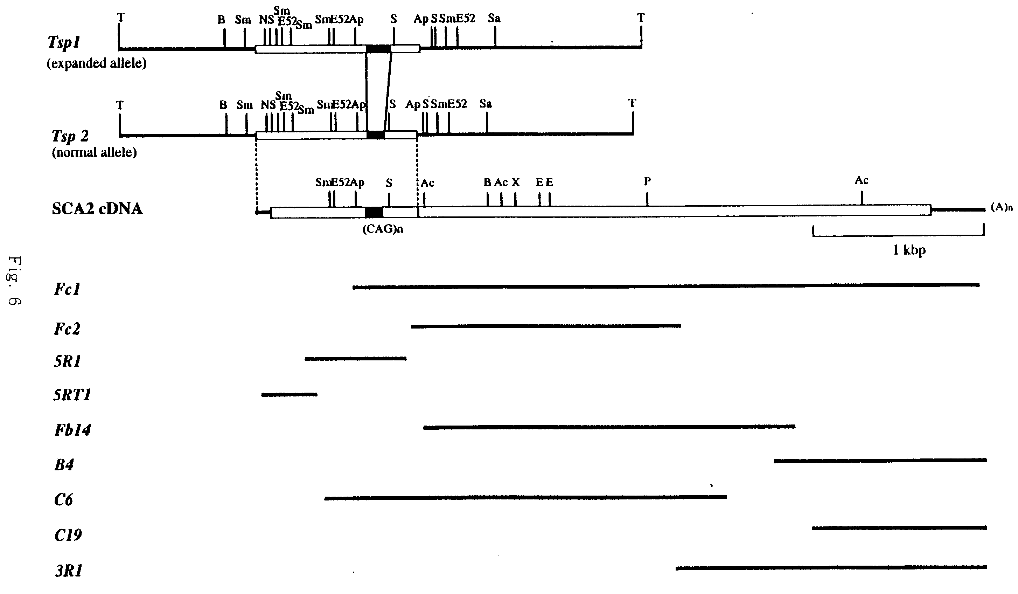

The SmaI-ApaI fragment (630 bp) of Tsp2 was sequenced and oligonucleotides F-1 (5'-CCC TCA CCA TGT CGC TGA AGC-3') and R-1 (5'-CGA CGC TAG AAG GCC GCT G-3') were designed such that the CAG repeat units are sandwiched between the oligonucleotides (see Fig. 1). Using oligonucleotides F-1 and R-1 as probes, human procephalic cortex cDNA library (STRATAGENE) was screened. Hybridization was performed in a solution containing 6 x SSC, 10 x Denhardt's solution, 0.5% SDS, 0.05% sodium pyrophosphate, 100 ng/ml of sheared salmon sperm DNA and end-labeled oligonucleotide probes at 55°C for 18 hours. After the hybridization, the membrane was finally washed with 6 x SSC containing 0.5% SDS and 0.05% sodium pyrophosphate at 55°C for 0.5 hours. A cDNA clone Fc1 with a size of 4.0 kb which hybridized with the both probes was obtained. The nucleotide sequences of Fc1, Tsp1 and Tsp2 were determined and compared. As a result, the nucleotide sequences in the vicinities of the CAG repeat units were identical except for the number of the CAG repeat units. Restriction maps of Tsp1 and Tsp2, as well as the sizes and positions of Fc1 and other fragments hereinbelow described, are shown in Fig. 6. Using Fc1 or a fragment isolated by the screening later described as a probe, human cDNA libraries (human procephalic cortex, human fetal brain, human brain and brain stem) were screened to isolate cDNA clones Fc2, Fb14, B4, C6 and C19 (see Fig. 6). To identify the 5'-end of Fc1, 5'-RACE (Frohman, M.A. et al, Proc. Natl. Acad. Sci. USA 85, 8998-9002 (1988)) was performed using 5'-RACE-Ready cDNA (Clonetech, Palo Alto, CA, USA). Primer R-1 was used for the first PCR, and Primer R-2 (5'-CTT GCG GAC ATT GGC AGC C-3', see Fig. 1) was used for the second PCR. In both PCRs, F-1 (see Fig. 1) was used as the forward primer. The 5'-RACE product (5R1) having the size of 350 bp was subcloned into pT7Blue T vector (pT7Blue T-vector (5R1)). The identification of 5R1 was confirmed by the overlapping with the nucleotide sequences of Fc1, Tsp1 and Tsp2. To identify the 3'-end of the cDNA, 3'-RACE was performed using 1 µg of poly(A)⁺mRNA extracted from human brain as a template and Primer F-13 (5'-TTC TCT CAG CCA AAG CCT TCT ACT ACC-3', see Fig. 3) as a primer. The obtained 3'-RACE product (3R1) with a size of 1300 bp was subcloned into pT7Blue T vector (pT7Blue T-vector (3R1)).

To investigate the 5'-end region of the cDNA, reverse transcription PCR (RT-PCR) was performed. That is, total RNAs extracted from an autopsy from human brain were digested by RNase-free DNase (PROMEGA) (Onodera, O. et al., Am. J. Hum. Geent. 57, 1050-1060(1995)). As the primers for the PCR, F1006 (5'-TAT CCG CAG CTC CGC TCC C-3', see Fig. 1) and R1002 (5'-AGC CGG GCC GAA ACG CGC CG-3') were used. PCR was performed in a solution with a total volume of 20 µM, which contained 5 pmol each of the each primer, 10 mM Tris HCl (pH8.3), 50 mM KCl, 1.5 mM MgCl₂, 1.7M N,N,N-trimethylglycine, 200 µM each of dATP, dCTP and TTP, 100 µM of dGTP, 100 µM of 7-deaza dGTP and 2.5 U of Taq polymerase (TAKARA SHUZO). After carrying out the initial denaturation at 96°C for 2 minutes, a cycle of a denaturation step at 96°C for 1 minute, an annealing step at 65°C for 1 minute and an extension step at 72°C for 1 minute were repeated 30 times, and a final extension step at 72°C for 5 minutes was performed, thereby carrying out the PCR. As a result, a clone 5R1 which extends upstream of 5R1 by 246 bp was obtained (see Fig. 6).

In Fig. 6, the hollow regions in the Tsp1 and Tsp2 fragments indicate the regions which exist in SCA2 cDNAs. The hollow regions in the SCA2 cDNA shows coding regions. The CAG repeating regions are shown as solid boxes. Restriction sites TspE1 (T) 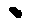 NotI (N) 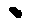 Sac II (S) 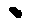 Sau3AI (Sa) 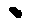 Sma I (Sm) 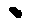 Eco52I (E52) 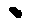 Apa I (Ap) 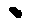 AccI (Ac) 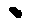 BamHI (B) 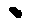 XhoI (X) 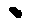 EcoRI (E) and Pst I (P) are shown. The size and position of each cDNA clone are shown below the consensus SCA2 cDNA.

In this example, nucleotide sequences of double-stranded DNAs were determined by the dideoxynucleotide chain termination method (Sanger, F. et al. Proc. Natl. Acad. Sci. USA 74, 5463-5467(1977); Chen E.Y. et al, DNA 4, 165-170 (1985)) using a double-stranded plasmid DNA as a template. To determine the nucleotide sequences of the CAG repeating regions and their flanking regions, genomic fragments containing the CAG repeating regions were amplified by PCR using biotinylated F-1 and RS-1 (5'-CCT CGG TGT CGC GGC GAC TTC C-3'). PCR was performed in a solution with a total volume of 25 µl, which contained 0.25 µM each of the each primer, 10 mM Tris HCl (pH8.3), 50 mM KCl, 2.0 mM MgCl₂, 1.7M N,N,N-trimethylglycine, 200 µM each of dNTP, 200 ng of the genomic DNA and 1.25 U of Taq polymerase (TAKARA SHUZO). After carrying out initial denaturation at 95°C for 1 minute, a cycle of a denaturation step at 95°C for 2 minutes, an annealing step at 62°C for 1 minute and an extension step at 72°C for 1 minute was repeated 32 times, and a final extension step at 72°C for 5 minutes was performed, thereby carrying out the PCR. Biotinylated chains were recovered using streptavidin-coated magnetic beads and were directly sequenced.

Based on the nucleotide sequences of the above-mentioned cDNA clones, a consensus SCA2 cDNA sequence with a length of 4351 bp excluding the poly A tail was determined (SEQ ID NO:1, Figs. 1-4, see Fig. 6). In SEQ ID NO: 1, the region from 4352nt to 4367nt is the poly A tail, and the number of "A" is not restricted to that shown in SEQ ID NO: 1. It was confirmed that the poly A tail exists at the same location in C19, B4 and 3R1 which were independent cDNA clones.

### Example 2 Measurement of CAG Repeat Units in Sample

Numbers of CAG repeat units were determined by polyacrylamide gel electrophoresis analysis of PCR products obtained using the primer pair of F-1 and R-1. PCR was performed in a total volume of 10 µl containing 10 mM Tris-HCl, pH 8.3, 50 mM KCl, 2.0 mM MgCl₂, 1.7 M *N,N,N*-trimethylglycine, 111KBq of [α-³²P]dCTP (111 Tbq/mmol), 30 µM dCTP, and 200 µM each of dATP, dGTP and TTP, 0.25 µM each of the two primers, 200 ng of genomic DNA and 1.25 U of Taq DNA polymerase. After an initial 2-min denaturation at 95°C, PCR was performed for 32 cycles of 1-min denaturation at 95°C, 1-min annealing at 60°C and 1-min extension at 72°C, followed by a final extension at 72°C for 5 min. Sequence ladders obtained using the cloned genomic segments of the SCA2 gene, which contain various sizes of CAG repeats, were used as size markers. For normal alleles containing one or two CAA interruptions, the numbers of the CAA units were included in the CAG repeat size. For SCA2 alleles having expanded CAG region, the above-mentioned insert sequence immediately after the CAG region was not included in the size of the CAG region.

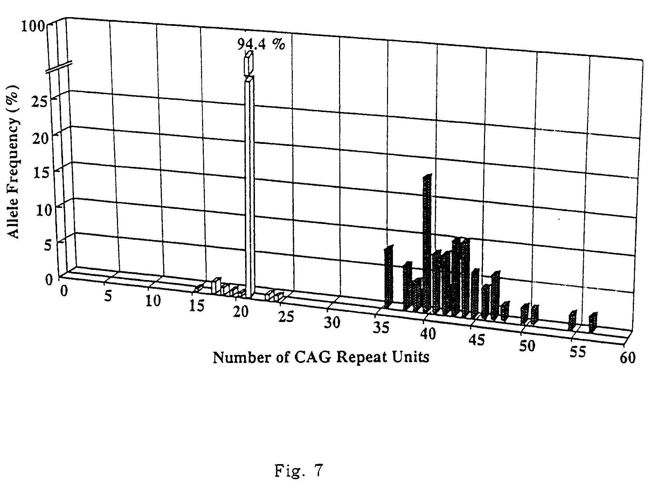

By the above-described method, the numbers of the CAG repeat units of normal individuals (286 chromosomes) and 10 pedigrees of SCA2 patients (34 SCA2 chromosomes) were determined. The results are shown in Fig. 7. In Fig. 7, open bars indicate the results of the normal genes and solid bars indicate the results of the SCA2 genes.

As is apparent from Fig. 7, in all of the normal genes, the numbers of the CAG repeat units were not more than 24, while in all of the SCA2 genes, they were not less than 35. Thus, it was confirmed that the cDNA identified as described above is the cDNA of the causative gene of SCA2.

SEQUENCE LISTING

SEQ ID NO: 1

SEQUENCE LENGTH: 4367

SEQUENCE TYPE: nucleic acid

STRNDEDNESS: double

TOPOLOGY: linear

SEQUENCE DESCRIPTION

TATCCGCACC TCCGCTCCCA CCCGGCGCCT CGGCGCGCCC GCCCTCCG ATG CGC TCA 57

Met Arg Ser

1

GCG GCC GCA GCT CCT CGG AGT CCC GCG GTG GCC ACC GAG TCT CGC CGC 105

Ala Ala Ala Ala Pro Arg Ser Pro Ala Val Ala Thr Glu Ser Arg Arg

    5                   10                  15

TTC GCC GCA GCC AGG TGG CCC GGG TGG CGC TCG CTC CAG CGG CCG GCG 153

Phe Ala Ala Ala Arg Trp Pro Gly Trp Arg Ser Leu Gln Arg Pro Ala

20                  25                  30                  35

CGG CGG AGC GGG CGG GGC GGC GGT GGC GCG GCC CCG GGA CCG TAT CCC 201

Arg Arg Ser Gly Arg Gly Gly Gly Gly Ala Ala Pro Gly Pro Tyr Pro

                40                  45                  50

TCC GCC GCC CCT CCC CCG CCC GGC CCC GGC CCC CCT CCC TCC CGG CAG 249

Ser Ala Ala Pro Pro Pro Pro Gly Pro Gly Pro Pro Pro Ser Arg Gln

            55                  60                  65

AGC TCG CCT CCC TCC GCC TCA GAC TGT TTT GGT AGC AAC GGC AAC GGC 297

Ser Ser Pro Pro Ser Ala Ser Asp Cys Phe Gly Ser Asn Gly Asn Gly

        70                  75                  80

GGC GGC GCG TTT CGG CCC GGC TCC CGG CGG CTC CTT GGT CTC GGC GGG 345

Gly Gly Ala Phe Arg Pro Gly Ser Arg Arg Leu Leu Gly Leu Gly Gly

85                    90                    95
CCT CCC CGC CCC TTC GTC GTC GTC CTT CTC CCC CTC GCC AGC CCG GGC    393
Pro Pro Arg Pro Phe Val Val Val Leu Leu Pro Leu Ala Ser Pro Gly
100                    105                    110                    115
GCC CCT CCG GCC GCG CCA ACC CGC GCC TCC CCG CTC GGC GCC CGT GCG    441
Ala Pro Pro Ala Ala Pro Thr Arg Ala Ser Pro Leu Gly Ala Arg Ala
                    120                    125                    130
TCC CCG CCG CGT TCC GGC GTC TCC TTG GCG CGC CCG GCT CCC GGC TGT    489
Ser Pro Pro Arg Ser Gly Val Ser Leu Ala Arg Pro Ala Pro Gly Cys
               135                    140                    145
CCC CGC CCG GCG TGC GAG CCG GTG TAT GGG CCC CTC ACC ATG TCG CTG    537
Pro Arg Pro Ala Cys Glu Pro Val Tyr Gly Pro Leu Thr Met Ser Leu
          150                    155                    160
AAG CCC CAG CAG CAG CAG CAG CAG CAG CAG CAG CAG CAG CAG CAG CAA    585
Lys Pro Gln Gln Gln Gln Gln Gln Gln Gln Gln Gln Gln Gln Gln Gln
     165                    170                    175
CAG CAG CAG CAG CAG CAG CAG CAG CAG CCG CCG CCC GCG GCT GCC AAT    633
Gln Gln Gln Gln Gln Gln Gln Gln Gln Pro Pro Pro Ala Ala Ala Asn
180                    185                    190                    195
GTC CGC AAG CCC GGC GGC AGC GGC CTT CTA GCG TCG CCC GCC GCC GCG    681
Val Arg Lys Pro Gly Gly Ser Gly Leu Leu Ala Ser Pro Ala Ala Ala
                    200                    205                    210
CCT TCG CCG TCC TCG TCC TCG GTC TCC TCG TCC TCG GCC ACG GCT CCC    729
Pro Ser Pro Ser Ser Ser Ser Val Ser Ser Ser Ser Ala Thr Ala Pro
               215                    220                    225
TCC TCG GTG GTC GCG GCG ACC TCC GGC GGC GGG AGG CCC GGC CTG GGC    777
Ser Ser Val Val Ala Ala Thr Ser Gly Gly Gly Arg Pro Gly Leu Gly
          230                    235                    240

AGA GGT CGA AAC AGT AAC AAA GGA CTG CCT CAG TCT ACG ATT TCT TTT 825
Arg Gly Arg Asn Ser Asn Lys Gly Leu Pro Gln Ser Thr Ile Ser Phe
    245                 250                 255
GAT GGA ATC TAT GCA AAT ATG AGG ATG GTT CAT ATA CTT ACA TCA GTT 873
Asp Gly Ile Tyr Ala Asn Met Arg Met Val His Ile Leu Thr Ser Val
260                 265                 270                 275
GTT GGC TCC AAA TGT GAA GTA CAA GTG AAA AAT GGA GGT ATA TAT GAA 921
Val Gly Ser Lys Cys Glu Val Gln Val Lys Asn Gly Gly Ile Tyr Glu
                280                 285                 290
GGA GTT TTT AAA ACT TAC AGT CCG AAG TGT GAT TTG GTA CTT GAT GCC 969
Gly Val Phe Lys Thr Tyr Ser Pro Lys Cys Asp Leu Val Leu Asp Ala
            295                 300                 305
GCA CAT GAG AAA AGT ACA GAA TCC AGT TCG GGG CCG AAA CGT GAA GAA 1017
Ala His Glu Lys Ser Thr Glu Ser Ser Ser Gly Pro Lys Arg Glu Glu
        310                 315                 320
ATA ATG GAG AGT ATT TTG TTC AAA TGT TCA GAC TTT GTT GTG GTA CAG 1065
Ile Met Glu Ser Ile Leu Phe Lys Cys Ser Asp Phe Val Val Val Gln
    325                 330                 335
TTT AAA GAT ATG GAC TCC AGT TAT GCA AAA AGA GAT GCT TTT ACT GAC 1113
Phe Lys Asp Met Asp Ser Ser Tyr Ala Lys Arg Asp Ala Phe Thr Asp
340                 345                 350                 355
TCT GCT ATC AGT GCT AAA GTG AAT GGC GAA CAC AAA GAG AAG GAC CTG 1161
Ser Ala Ile Ser Ala Lys Val Asn Gly Glu His Lys Glu Lys Asp Leu
                360                 365                 370
GAG CCC TGG GAT GCA GGT GAA CTC ACA GCC AAT GAG GAA CTT GAG GCT 1209
Glu Pro Trp Asp Ala Gly Glu Leu Thr Ala Asn Glu Glu Leu Glu Ala
            375                 380                 385
TTG GAA AAT GAC GTA TCT AAT GGA TGG GAT CCC AAT GAT ATG TTT CGA 1257

Leu Glu Asn Asp Val Ser Asn Gly Trp Asp Pro Asn Asp Met Phe Arg
        390                 395                 400
TAT AAT GAA GAA AAT TAT GGT GTA GTG TCT ACG TAT GAT AGC AGT TTA 1305
Tyr Asn Glu Glu Asn Tyr Gly Val Val Ser Thr Tyr Asp Ser Ser Leu
    405                 410                 415
TCT TCG TAT ACA GTG CCC TTA GAA AGA GAT AAC TCA GAA GAA TTT TTA 1353
Ser Ser Tyr Thr Val Pro Leu Glu Arg Asp Asn Ser Glu Glu Phe Leu
420                 425                 430                 435
AAA CGG GAA GCA AGG GCA AAC CAG TTA GCA GAA GAA ATT GAG TCA AGT 1401
Lys Arg Glu Ala Arg Ala Asn Gln Leu Ala Glu Glu Ile Glu Ser Ser
                440                 445                 450
GCC CAG TAC AAA GCT CGA GTG GCC CTG GAA AAC GAT GAT AGG AGT GAG 1449
Ala Gln Tyr Lys Ala Arg Val Ala Leu Glu Asn Asp Asp Arg Ser Glu
            455                 460                 465
GAA GAA AAA TAC ACA GCA GTT CAG AGA AAT TCC AGT GAA CGT GAG GGG 1497
Glu Glu Lys Tyr Thr Ala Val Gln Arg Asn Ser Ser Glu Arg Glu Gly
        470                 475                 480
CAC AGC ATA AAC ACT AGG GAA AAT AAA TAT ATT CCT CCT GGA CAA AGA 1545
His Ser Ile Asn Thr Arg Glu Asn Lys Tyr Ile Pro Pro Gly Gln Arg
    485                 490                 495
AAT AGA GAA GTC ATA TCC TGG GGA AGT GGG AGA CAG AAT TCA CCG CGT 1593
Asn Arg Glu Val Ile Ser Trp Gly Ser Gly Arg Gln Asn Ser Pro Arg
500                 505                 510                 515
ATG GGC CAG CCT GGA TCG GGC TCC ATG CCA TCA AGA TCC ACT TCT CAC 1641
Met Gly Gln Pro Gly Ser Gly Ser Met Pro Ser Arg Ser Thr Ser His
                520                 525                 530
ACT TCA GAT TTC AAC CCG AAT TCT GGT TCA GAC CAA AGA GTA GTT AAT 1689
Thr Ser Asp Phe Asn Pro Asn Ser Gly Ser Asp Gln Arg Val Val Asn

            535                 540                 545
GGA GGT GTT CCC TGG CCA TCG CCT TGC CCA TCT CCT TCC TCT CGC CCA 1737
Gly Gly Val Pro Trp Pro Ser Pro Cys Pro Ser Pro Ser Ser Arg Pro
        550                 555                 560
CCT TCT CGC TAC CAG TCA GGT CCC AAC TCT CTT CCA CCT CGG GCA GCC 1785
Pro Ser Arg Tyr Gln Ser Gly Pro Asn Ser Leu Pro Pro Arg Ala Ala
    565                 570                 575
ACC CCT ACA CGG CCG CCC TCC AGG CCC CCC TCG CGG CCA TCC AGA CCC 1833
Thr Pro Thr Arg Pro Pro Ser Arg Pro Pro Ser Arg Pro Ser Arg Pro
580                 585                 590                 595
CCG TCT CAC CCC TCT GCT CAT GGT TCT CCA GCT CCT GTC TCT ACT ATG 1881
Pro Ser His Pro Ser Ala His Gly Ser Pro Ala Pro Val Ser Thr Met
                600                 605                 610
CCT AAA CGC ATG TCT TCA GAA GGG CCT CCA AGG ATG TCC CCA AAG GCC 1929
Pro Lys Arg Met Ser Ser Glu Gly Pro Pro Arg Met Ser Pro Lys Ala
            615                 620                 625
CAG CGA CAT CCT CGA AAT CAC AGA GTT TCT GCT GGG AGG GGT TCC ATA 1977
Gln Arg His Pro Arg Asn His Arg Val Ser Ala Gly Arg Gly Ser Ile
        630                 635                 640
TCC AGT GGC CTA GAA TTT GTA TCC CAC AAC CCA CCC AGT GAA GCA GCT 2025
Ser Ser Gly Leu Glu Phe Val Ser His Asn Pro Pro Ser Glu Ala Ala
    645                 650                 655
ACT CCT CCA GTA GCA AGG ACC AGT CCC TCG GGG GGA ACG TGG TCA TCA 2073
Thr Pro Pro Val Ala Arg Thr Ser Pro Ser Gly Gly Thr Trp Ser Ser
660                 665                 670                 675
GTG GTC AGT GGG GTT CCA AGA TTA TCC CCT AAA ACT CAT AGA CCC AGG 2121
Val Val Ser Gly Val Pro Arg Leu Ser Pro Lys Thr His Arg Pro Arg
                680                 685                 690

TCT CCC AGA CAG AAC AGT ATT GGA AAT ACC CCC AGT GGG CCA GTT CTT 2169
Ser Pro Arg Gln Asn Ser Ile Gly Asn Thr Pro Ser Gly Pro Val Leu
            695                 700                 705
GCT TCT CCC CAA GCT GGT ATT ATT CCA ACT GAA GCT GTT GCC ATG CCT 2217
Ala Ser Pro Gln Ala Gly Ile Ile Pro Thr Glu Ala Val Ala Met Pro
        710                 715                 720
ATT CCA GCT GCA TCT CCT ACG CCT GCT AGT CCT GCA TCG AAC AGA GCT 2265
Ile Pro Ala Ala Ser Pro Thr Pro Ala Ser Pro Ala Ser Asn Arg Ala
    725                 730                 735
GTT ACC CCT TCT AGT GAG GCT AAA GAT TCC AGG CTT CAA GAT CAG AGG 2313
Val Thr Pro Ser Ser Glu Ala Lys Asp Ser Arg Leu Gln Asp Gln Arg
740                 745                 750                 755
CAG AAC TCT CCT GCA GGG AAT AAA GAA AAT ATT AAA CCC AAT GAA ACA 2361
Gln Asn Ser Pro Ala Gly Asn Lys Glu Asn Ile Lys Pro Asn Glu Thr
                760                 765                 770
TCA CCT AGC TTC TCA AAA GCT GAA AAC AAA GGT ATA TCA CCA GTT GTT 2409
Ser Pro Ser Phe Ser Lys Ala Glu Asn Lys Gly Ile Ser Pro Val Val
            775                 780                 785
TCT GAA CAT AGA AAA CAG ATT GAT GAT TTA AAG AAA TTT AAG AAT GAT 2457
Ser Glu His Arg Lys Gln Ile Asp Asp Leu Lys Lys Phe Lys Asn Asp
        790                 795                 800
TTT AGG TTA CAG CCA AGT TCT ACT TCT GAA TCT ATG GAT CAA CTA CTA 2505
Phe Arg Leu Gln Pro Ser Ser Thr Ser Glu Ser Met Asp Gln Leu Leu
    805                 810                 815
AAC AAA AAT AGA GAG GGA GAA AAA TCA AGA GAT TTG ATC AAA GAC AAA 2553
Asn Lys Asn Arg Glu Gly Glu Lys Ser Arg Asp Leu Ile Lys Asp Lys
820                 825                 830                 835
ATT GAA CCA AGT GCT AAG GAT TCT TTC ATT GAA AAT AGC AGC AGC AAC 2601

Ile Glu Pro Ser Ala Lys Asp Ser Phe Ile Glu Asn Ser Ser Ser Asn
                840                 845                 850
TGT ACC AGT GGC AGC AGC AAG CCG AAT AGC CCC AGC ATT TCC CCT TCA 2649
Cys Thr Ser Gly Ser Ser Lys Pro Asn Ser Pro Ser Ile Ser Pro Ser
            855                 860                 865
ATA CTT AGT AAC ACG GAG CAC AAG AGG GGA CCT GAG GTC ACT TCC CAA 2697
Ile Leu Ser Asn Thr Glu His Lys Arg Gly Pro Glu Val Thr Ser Gln
        870                 875                 880
GGG GTT CAG ACT TCC AGC CCA GCA TGT AAA CAA GAG AAA GAC GAT AAG 2745
Gly Val Gln Thr Ser Ser Pro Ala Cys Lys Gln Glu Lys Asp Asp Lys
    885                 890                 895
GAA GAG AAG AAA GAC GCA GCT GAG CAA GTT AGG AAA TCA ACA TTG AAT 2793
Glu Glu Lys Lys Asp Ala Ala Glu Gln Val Arg Lys Ser Thr Leu Asn
900                 905                 910                 915
CCC AAT GCA AAG GAG TTC AAC CCA CGT TCC TTC TCT CAG CCA AAG CCT 2841
Pro Asn Ala Lys Glu Phe Asn Pro Arg Ser Phe Ser Gln Pro Lys Pro
                920                 925                 930
TCT ACT ACC CCA ACT TCA CCT CGG CCT CAA GCA CAA CCT AGC CCA TCT 2889
Ser Thr Thr Pro Thr Ser Pro Arg Pro Gln Ala Gln Pro Ser Pro Ser
            935                 940                 945
ATG GTG GGT CAT CAA CAG CCA ACT CCA GTT TAT ACT CAG CCT GTT TGT 2937
Met Val Gly His Gln Gln Pro Thr Pro Val Tyr Thr Gln Pro Val Cys
        950                 955                 960
TTT GCA CCA AAT ATG ATG TAT CCA GTC CCA GTG AGC CCA GGC GTG CAA 2985
Phe Ala Pro Asn Met Met Tyr Pro Val Pro Val Ser Pro Gly Val Gln
    965                 970                 975
CCT TTA TAC CCA ATA CCT ATG ACG CCC ATG CCA GTG AAT CAA GCC AAG 3033
Pro Leu Tyr Pro Ile Pro Met Thr Pro Met Pro Val Asn Gln Ala Lys

980                 985                 990                 995
ACA TAT AGA GCA GTA CCA AAT ATG CCC CAA CAG CGG CAA GAC CAG CAT 3081
Thr Tyr Arg Ala Val Pro Asn Met Pro Gln Gln Arg Gln Asp Gln His
                1000                1005                1010
CAT CAG AGT GCC ATG ATG CAC CCA GCG TCA GCA GCG GGC CCA CCG ATT 3129
His Gln Ser Ala Met Met His Pro Ala Ser Ala Ala Gly Pro Pro Ile
            1015                1020                1025
GCA GCC ACC CCA CCA GCT TAC TCC ACG CAA TAT GTT GCC TAC AGT CCT 3177
Ala Ala Thr Pro Pro Ala Tyr Ser Thr Gln Tyr Val Ala Tyr Ser Pro
        1030                1035                1040
CAG CAG TTC CCA AAT CAG CCC CTT GTT CAG CAT GTG CCA CAT TAT CAG 3225
Gln Gln Phe Pro Asn Gln Pro Leu Val Gln His Val Pro His Tyr Gln
    1045                1050                1055
TCT CAG CAT CCT CAT GTC TAT AGT CCT GTA ATA CAG GGT AAT GCT AGA 3273
Ser Gln His Pro His Val Tyr Ser Pro Val Ile Gln Gly Asn Ala Arg
1060                1065                1070                1075
ATG ATG GCA CCA CCA ACA CAC GCC CAG CCT GGT TTA GTA TCT TCT TCA 3321
Met Met Ala Pro Pro Thr His Ala Gln Pro Gly Leu Val Ser Ser Ser
                1080                1085                1090
GCA ACT CAG TAC GGG GCT CAT GAG CAG ACG CAT GCG ATG TAT GCA TGT 3369
Ala Thr Gln Tyr Gly Ala His Glu Gln Thr His Ala Met Tyr Ala Cys
            1095                1100                1105
CCC AAA TTA CCA TAC AAC AAG GAG ACA AGC CCT TCT TTC TAC TTT GCC 3417
Pro Lys Leu Pro Tyr Asn Lys Glu Thr Ser Pro Ser Phe Tyr Phe Ala
        1110                1115                1120
ATT TCC ACG GGC TCC CTT GCT CAG CAG TAT GCG CAC CCT AAC GCT ACC 3465
Ile Ser Thr Gly Ser Leu Ala Gln Gln Tyr Ala His Pro Asn Ala Thr
    1125                1130                1135

CTG CAC CCA CAT ACT CCA CAC CCT CAG CCT TCA GCT ACC CCC ACT GGA 3513
Leu His Pro His Thr Pro His Pro Gln Pro Ser Ala Thr Pro Thr Gly
1140                1145                1150                1155
CAG CAG CAA AGC CAA CAT GGT GGA AGT CAT CCT GCA CCC AGT CCT GTT 3561
Gln Gln Gln Ser Gln His Gly Gly Ser His Pro Ala Pro Ser Pro Val
                1160                1165                1170
CAG CAC CAT CAG CAC CAG GCC GCC CAG GCT CTC CAT CTG GCC AGT CCA 3609
Gln His His Gln His Gln Ala Ala Gln Ala Leu His Leu Ala Ser Pro
            1175                1180                1185
CAG CAG CAG TCA GCC ATT TAC CAC GCG GGG CTT GCG CCA ACT CCA CCC 3657
Gln Gln Gln Ser Ala Ile Tyr His Ala Gly Leu Ala Pro Thr Pro Pro
        1190                1195                1200
TCC ATG ACA CCT GCC TCC AAC ACG CAG TCG CCA CAG AAT AGT TTC CCA 3705
Ser Met Thr Pro Ala Ser Asn Thr Gln Ser Pro Gln Asn Ser Phe Pro
    1205                1210                1215
GCA GCA CAA CAG ACT GTC TTT ACG ATC CAT CCT TCT CAC GTT CAG CCG 3753
Ala Ala Gln Gln Thr Val Phe Thr Ile His Pro Ser His Val Gln Pro
1220                1225                1230                1235
GCG TAT ACC AAC CCA CCC CAC ATG GCC CAC GTA CCT CAG GCT CAT GTA 3801
Ala Tyr Thr Asn Pro Pro His Met Ala His Val Pro Gln Ala His Val
                1240                1245                1250
CAG TCA GGA ATG GTT CCT TCT CAT CCA ACT GCC CAT GCG CCA ATG ATG 3849
Gln Ser Gly Met Val Pro Ser His Pro Thr Ala His Ala Pro Met Met
            1255                1260                1265
CTA ATG ACG ACA CAG CCA CCC GGC GGT CCC CAG GCC GCC CTC GCT CAA 3897
Leu Met Thr Thr Gln Pro Pro Gly Gly Pro Gln Ala Ala Leu Ala Gln
        1270                1275                1280
AGT GCA CTA CAG CCC ATT CCA GTC TCG ACA ACA GCG CAT TTC CCC TAT 3945

Ser Ala Leu Gln Pro Ile Pro Val Ser Thr Thr Ala His Phe Pro Tyr
    1285                1290                1295
ATG ACG CAC CCT TCA GTA CAA GCC CAC CAC CAA CAG CAG TTG 3987
Met Thr His Pro Ser Val Gln Ala His His Gln Gln Gln Leu
1300                1305                1310
TAAGGCTGCC CTGGAGGAAC CGAAAGGCCA AATTCCCTCC TCCCTTCTAC TGCTTCTACC 4047
AACTGGAAGC ACAGAAAACT AGAATTTCAT TTATTTTGTT TTTAAAATAT ATATGTTGAT 4107
TTCTTGTAAC ATCCAATAGG AATGCTAACA GTTCACTTGC AGTGGAAGAT ACTTGGACCG 4167
AGTAGAGGCA TTTAGGAACT TGGGGGCTAT TCCATAATTC CATATGCTGT TTCAGAGTCC 4227
CGCAGGTACC CCAGCTCTGC TTGCCGAAAC TGGAAGTTAT TTATTTTTTA ATAACCCTTG 4287
AAAGTCATGA ACACATCAGC TAGCAAAAGA AGTAACAAGA GTGATTCTTG CTGCTATTAC 4347
TGCTAAAAAA AAAAAAAAAA 4367

SEQ ID NO: 2
SEQUENCE LENGTH: 203
SEQUENCE TYPE: nucleic acid
STRNDEDNESS: single
TOPOLOGY: linear
SEQUENCE DESCRIPTION
CACCACCAGC AACAGCAACA GCAGCAGCAG CAGCAGCAGC AGCAGCAGCA GCAGCAGCAG 60
CAGCAGCAGC AGCAGCAGCA GCAGCAGCAG CAGCAGCAGC AGCAGCAGCA GCAGCAGCAG 120
CAGCAGCAGC AGCAGCAGCA GCAGCAGCAG CAGCAGCAGC AGCAGCAGCA GCAGCAGCAG 180
CAGCATCACG GAAACTCTGG GCC 203

SEQ ID NO: 3
SEQUENCE LENGTH: 20
SEQUENCE TYPE: nucleic acid

STRNDEDNESS: single

TOPOLOGY: linear

SEQUENCE DESCRIPTION

CACCACCAGC AACAGCAACA 20

SEQ ID NO: 4

SEQUENCE LENGTH: 20

SEQUENCE TYPE: nucleic acid

STRNDEDNESS: single

TOPOLOGY: linear

SEQUENCE DESCRIPTION

GGCCCAGAGT TTCCGTGATG 20

SEQ ID NO: 5

SEQUENCE LENGTH: 165

SEQUENCE TYPE: nucleic acid

STRNDEDNESS: single

TOPOLOGY: linear

SEQUENCE DESCRIPTION

CAGCAGCAGC AGCAGCAGCA GCAGCAGCAG CAGCAGCAGC AGCAGCAGCA GCAGCAGCAG 60

CAGCAGCAGC AGCAGCAGCA GCAGCAGCAG CAGCAGCAGC AGCAGCAGCA GCAGCAGCAG 120

CAGCAGCAGC AGCAGCAGCA GCAGCAGCAG CAGCAGCAGC AGCAG 165

SEQ ID NO: 6

SEQUENCE LENGTH: 21

SEQUENCE TYPE: nucleic acid

STRNDEDNESS: single

TOPOLOGY: linear
SEQUENCE DESCRIPTION
CCCTCACCAT GTCGCTGAAG C 21

SEQ ID NO: 7
SEQUENCE LENGTH: 19
SEQUENCE TYPE: nucleic acid
STRNDEDNESS: single
TOPOLOGY: linear
SEQUENCE DESCRIPTION
CGACGCTAGA AGGCCGCTG 19

SEQ ID NO: 8
SEQUENCE LENGTH: 19
SEQUENCE TYPE: nucleic acid
STRNDEDNESS: single
TOPOLOGY: linear
SEQUENCE DESCRIPTION
CTTGCGGACA TTGGCAGCC 19

SEQ ID NO: 9
SEQUENCE LENGTH: 27
SEQUENCE TYPE: nucleic acid
STRNDEDNESS: single
TOPOLOGY: linear
SEQUENCE DESCRIPTION
TTCTCTCAGC CAAAGCCTTC TACTACC 27

SEQ ID NO: 10

SEQUENCE LENGTH: 19

SEQUENCE TYPE: nucleic acid

STRNDEDNESS: single

TOPOLOGY: linear

SEQUENCE DESCRIPTION

TATCCGCAGC TCCGCTCCC 19

SEQ ID NO: 11

SEQUENCE LENGTH: 20

SEQUENCE TYPE: nucleic acid

STRNDEDNESS: single

TOPOLOGY: linear

SEQUENCE DESCRIPTION

AGCCGGGCCG AAACGCGCCG 20

## Claims

1. A nucleic acid fragment comprising a nucleic acid region encoding an amino acid sequence shown in SEQ ID NO: 1 (provided that the number of repeat units of Gln from the 166th to 188th amino acid varies between 15 and 100).

2. The nucleic acid fragment according to claim 1, wherein said nucleic acid region is the region from 49nt to 3987nt (provided that the number of repeat units of CAG or CAA in the region from the 543nt to 612nt varies between 15 and 100, and that the CAA in this region may be CAG).

3. A protein having the amino acid sequence encoded by said nucleic acid fragment according to claim 1 or 2.

4. An antibody which undergoes antigen-antibody reaction with said protein according to claim 3.

5. An antisense nucleic acid having a size of not less than 15 bp, which hybridizes with the mRNA transcribed from the nucleic acid fragment according to claim 1 or 2 so as to inhibit translation thereof.

6. A recombinant vector comprising said nucleic acid fragment according to claim 1 or 2 incorporated into an expression vector which can express a desired gene in human body, which recombinant vector can express said nucleic acid fragment in human body.

7. A method comprising introducing said recombinant vector according to claim 6 into human body and expressing said nucleic acid fragment according to claim 1 or 2 in said human body.
